# EUROPÄISCHE PATENTANMELDUNG

(11) **EP 2 487 237 A1**
(43) Veröffentlichungstag der Anmeldung: **15.08.2012**
(21) Anmeldenummer: 12155383.8
(22) Anmeldetag: 14.02.2012
(51) Int. Cl.: C12N 9/58, C12N 9/24

(54) **Bifunktionales Polypeptid und Zusammensetzungen dieses enthaltend**

(30) Priorität: 14.02.2011 DE 102011000701
(71) Anmelder: Gau, Achim, 32423 Minden (DE); Ramadan, Mohamed, Assiut 71516 (EG)
(72) Erfinder: Gau, Achim, 32423 Minden (DE); Ramadan, Mohamed, Assiut 71516 (EG)
(74) Vertreter: Kröncke, Rolf

(57) **Zusammenfassung**

Die vorliegende Anmeldung betrifft isolierte bifunktionale Polypeptide. Genauer betrifft die vorliegende Anmeldung Polypeptide mit einer Glucanaseaktivität und einer Proteaseaktivität, wobei das Polypeptid ein Derivat eines natürlich vorkommenden Polypeptids mit Glucanase- und gleichzeitig einer Proteaseaktivität ist. In einem weiteren Aspekt werden Zusammensetzungen bereitgestellt dieses Polypeptid enthaltend. Schließlich richtet sich die vorliegende Anmeldung auf die Verwendung der erfindungsgemäßen Polypeptide in Wasch- oder Reinigungsmitteln, als antimikrobielles Mittel oder zum Aufschluss von tierischem, pflanzlichem und mikrobiellem Material sowie entsprechende Verfahren.

SEQUENCE LISTING

<110>  Gottfried Wilhelm Leibniz Universität Hannover
       Gau, Achim
       Abd-Elghany Osman Mohamed, Ramadan

<120>  Bifunktionales Polypeptid und Zusammensetzungen dieses enthaltend

<130>  2674-086-EP-1

<150>  DE102011000701
<151>  2011-02-11

<160>  6

<170>  PatentIn version 3.5

<210>  1
<211>  981
<212>  DNA
<213>  Venturia inaequalis


<220>
<221>  CDS
<222>  (1)..(981)

<400>  1
atg ttc gca aaa ctt ctc gcc ctg gca gca ttg cct gct gct tcc aac        48
Met Phe Ala Lys Leu Leu Ala Leu Ala Ala Leu Pro Ala Ala Ser Asn
1               5                   10                  15

gca ttt gga gtc ctc aag ggt ttc aac tac ggc tcg act gat gcc tct        96
Ala Phe Gly Val Leu Lys Gly Phe Asn Tyr Gly Ser Thr Asp Ala Ser
            20                  25                  30

gga gtt gtc aag gac cag gcc cgt ttc gag caa gaa ttc agc acc gct       144
Gly Val Val Lys Asp Gln Ala Arg Phe Glu Gln Glu Phe Ser Thr Ala
        35                  40                  45

cag aac ttg gtt gga acc agt gga ttc aac agt gcc aga cta tac aca       192
Gln Asn Leu Val Gly Thr Ser Gly Phe Asn Ser Ala Arg Leu Tyr Thr
    50                  55                  60

aca atc caa gga gga acc acg aat agc cca atc tca gca att cca gct       240
Thr Ile Gln Gly Gly Thr Thr Asn Ser Pro Ile Ser Ala Ile Pro Ala
65                  70                  75                  80

gcc atc agc acc cag acc tct ctc ctt ctc gga atc tgg aca tcc gca       288
Ala Ile Ser Thr Gln Thr Ser Leu Leu Leu Gly Ile Trp Thr Ser Ala
                85                  90                  95

ggc caa gcc att gtg gac aac gag att gcg gca ttg aag gct gct atc       336
Gly Gln Ala Ile Val Asp Asn Glu Ile Ala Ala Leu Lys Ala Ala Ile
            100                 105                 110

agt cag tac ggc aca gct ttc act gac ctg att gtt gcc gtt tct gtt       384
Ser Gln Tyr Gly Thr Ala Phe Thr Asp Leu Ile Val Ala Val Ser Val
        115                 120                 125

ggt tct gag gat ctt tac cgt aac tct gga tac cca gga gct tca gac       432
Gly Ser Glu Asp Leu Tyr Arg Asn Ser Gly Tyr Pro Gly Ala Ser Asp
    130                 135                 140

cct ggt cca ggt gcc aat cct gat gtt ctt gcc aac tac att gga caa       480
Pro Gly Pro Gly Ala Asn Pro Asp Val Leu Ala Asn Tyr Ile Gly Gln
145                 150                 155                 160

gtc aag gcg gcc att gct ggc aca tct gcg gag gga cga ctg gtc gga          528
Val Lys Ala Ala Ile Ala Gly Thr Ser Ala Glu Gly Arg Leu Val Gly
                165                 170                 175

cat gtg gat acc tgg act gcc ttc gtc aac agc tcc aac aac gca ctc          576
His Val Asp Thr Trp Thr Ala Phe Val Asn Ser Ser Asn Asn Ala Leu
            180                 185                 190

atc tcc gcc gcc gac ttc ttg ggt gtt gat gcc tac cca tac tac gag          624
Ile Ser Ala Ala Asp Phe Leu Gly Val Asp Ala Tyr Pro Tyr Tyr Glu
        195                 200                 205

tct gcc aac gga aac gac atc agt aac gca gcc aac ttg ttc gca agc          672
Ser Ala Asn Gly Asn Asp Ile Ser Asn Ala Ala Asn Leu Phe Ala Ser
    210                 215                 220

gca tac tcc cag gtc gtt gct gtg gca caa gga aaa cca gtc tgg gtt          720
Ala Tyr Ser Gln Val Val Ala Val Ala Gln Gly Lys Pro Val Trp Val
225                 230                 235                 240

acc gag gct gga tgg cca gtt tct gga cca aca gtg gca caa gct gta          768
Thr Glu Ala Gly Trp Pro Val Ser Gly Pro Thr Val Ala Gln Ala Val
                245                 250                 255

gct tcc cct gaa aat gcc cgc tca ttc tgg acc tcc gtt ggc tgc aac          816
Ala Ser Pro Glu Asn Ala Arg Ser Phe Trp Thr Ser Val Gly Cys Asn
            260                 265                 270

caa ctt ttc gat aag atc aat gtc tgg tgg ttc caa ttg gat gac tac          864
Gln Leu Phe Asp Lys Ile Asn Val Trp Trp Phe Gln Leu Asp Asp Tyr
        275                 280                 285

ccc act tca ccc aac cct gcg ttc ggt gtt ata ggc aca gcc ttt tcc          912
Pro Thr Ser Pro Asn Pro Ala Phe Gly Val Ile Gly Thr Ala Phe Ser
    290                 295                 300

acc act cct ctg ttc gat ctt tcc tgc cct gct acc acg aag tct cgt          960
Thr Thr Pro Leu Phe Asp Leu Ser Cys Pro Ala Thr Thr Lys Ser Arg
305                 310                 315                 320

agg aga tcc aac cgc gca gcc                                              981
Arg Arg Ser Asn Arg Ala Ala
                325


<210>  2
<211>  327
<212>  PRT
<213>  Venturia inaequalis

<400>  2

Met Phe Ala Lys Leu Leu Ala Leu Ala Ala Leu Pro Ala Ala Ser Asn
1               5                   10                  15


Ala Phe Gly Val Leu Lys Gly Phe Asn Tyr Gly Ser Thr Asp Ala Ser
            20                  25                  30


Gly Val Val Lys Asp Gln Ala Arg Phe Glu Gln Glu Phe Ser Thr Ala
        35                  40                  45


Gln Asn Leu Val Gly Thr Ser Gly Phe Asn Ser Ala Arg Leu Tyr Thr
    50                  55                  60

Thr Ile Gln Gly Gly Thr Thr Asn Ser Pro Ile Ser Ala Ile Pro Ala
65                  70                  75                  80

Ala Ile Ser Thr Gln Thr Ser Leu Leu Leu Gly Ile Trp Thr Ser Ala
                85                  90                  95

Gly Gln Ala Ile Val Asp Asn Glu Ile Ala Ala Leu Lys Ala Ala Ile
            100                 105                 110

Ser Gln Tyr Gly Thr Ala Phe Thr Asp Leu Ile Val Ala Val Ser Val
        115                 120                 125

Gly Ser Glu Asp Leu Tyr Arg Asn Ser Gly Tyr Pro Gly Ala Ser Asp
    130                 135                 140

Pro Gly Pro Gly Ala Asn Pro Asp Val Leu Ala Asn Tyr Ile Gly Gln
145                 150                 155                 160

Val Lys Ala Ala Ile Ala Gly Thr Ser Ala Glu Gly Arg Leu Val Gly
                165                 170                 175

His Val Asp Thr Trp Thr Ala Phe Val Asn Ser Ser Asn Asn Ala Leu
            180                 185                 190

Ile Ser Ala Ala Asp Phe Leu Gly Val Asp Ala Tyr Pro Tyr Tyr Glu
        195                 200                 205

Ser Ala Asn Gly Asn Asp Ile Ser Asn Ala Ala Asn Leu Phe Ala Ser
    210                 215                 220

Ala Tyr Ser Gln Val Val Ala Val Ala Gln Gly Lys Pro Val Trp Val
225                 230                 235                 240

Thr Glu Ala Gly Trp Pro Val Ser Gly Pro Thr Val Ala Gln Ala Val
                245                 250                 255

Ala Ser Pro Glu Asn Ala Arg Ser Phe Trp Thr Ser Val Gly Cys Asn
            260                 265                 270

Gln Leu Phe Asp Lys Ile Asn Val Trp Trp Phe Gln Leu Asp Asp Tyr
        275                 280                 285

Pro Thr Ser Pro Asn Pro Ala Phe Gly Val Ile Gly Thr Ala Phe Ser
    290                 295                 300

Thr Thr Pro Leu Phe Asp Leu Ser Cys Pro Ala Thr Thr Lys Ser Arg
305                 310                 315                 320

Arg Arg Ser Asn Arg Ala Ala
                325

<210>  3
<211>  762
<212>  DNA
<213>  Venturia inaequalis


<220>
<221>  CDS
<222>  (1)..(762)

<400>  3
aac tac ggc tcg act gat gcc tct gga gtt gtc aag gac cag gcc cgt        48
Asn Tyr Gly Ser Thr Asp Ala Ser Gly Val Val Lys Asp Gln Ala Arg
1               5                   10                  15

ttc gag caa gaa ttc agc acc gct cag aac ttg gtt gga acc agt gga        96
Phe Glu Gln Glu Phe Ser Thr Ala Gln Asn Leu Val Gly Thr Ser Gly
            20                  25                  30

ttc aac agt gcc aga cta tac aca aca atc caa gga gga acc acg aat       144
Phe Asn Ser Ala Arg Leu Tyr Thr Thr Ile Gln Gly Gly Thr Thr Asn
        35                  40                  45

agc cca atc tca gca att cca gct gcc atc agc acc cag acc tct ctc       192
Ser Pro Ile Ser Ala Ile Pro Ala Ala Ile Ser Thr Gln Thr Ser Leu
    50                  55                  60

ctt ctc gga atc tgg aca tcc gca ggc caa gcc att gtg gac aac gag       240
Leu Leu Gly Ile Trp Thr Ser Ala Gly Gln Ala Ile Val Asp Asn Glu
65                  70                  75                  80

att gcg gca ttg aag gct gct atc agt cag tac ggc aca gct ttc act       288
Ile Ala Ala Leu Lys Ala Ala Ile Ser Gln Tyr Gly Thr Ala Phe Thr
                85                  90                  95

gac ctg att gtt gcc gtt tct gtt ggt tct gag gat ctt tac cgt aac       336
Asp Leu Ile Val Ala Val Ser Val Gly Ser Glu Asp Leu Tyr Arg Asn
            100                 105                 110

tct gga tac cca gga gct tca gac cct ggt cca ggt gcc aat cct gat       384
Ser Gly Tyr Pro Gly Ala Ser Asp Pro Gly Pro Gly Ala Asn Pro Asp
        115                 120                 125

gtt ctt gcc aac tac att gga caa gtc aag gcg gcc att gct ggc aca       432
Val Leu Ala Asn Tyr Ile Gly Gln Val Lys Ala Ala Ile Ala Gly Thr
    130                 135                 140

tct gcg gag gga cga ctg gtc gga cat gtg gat acc tgg act gcc ttc       480
Ser Ala Glu Gly Arg Leu Val Gly His Val Asp Thr Trp Thr Ala Phe
145                 150                 155                 160

gtc aac agc tcc aac aac gca ctc atc tcc gcc gcc gac ttc ttg ggt       528
Val Asn Ser Ser Asn Asn Ala Leu Ile Ser Ala Ala Asp Phe Leu Gly
                165                 170                 175

gtt gat gcc tac cca tac tac gag tct gcc aac gga aac gac atc agt       576
Val Asp Ala Tyr Pro Tyr Tyr Glu Ser Ala Asn Gly Asn Asp Ile Ser
            180                 185                 190

aac gca gcc aac ttg ttc gca agc gca tac tcc cag gtc gtt gct gtg       624
Asn Ala Ala Asn Leu Phe Ala Ser Ala Tyr Ser Gln Val Val Ala Val
        195                 200                 205

gca caa gga aaa cca gtc tgg gtt acc gag gct gga tgg cca gtt tct       672
Ala Gln Gly Lys Pro Val Trp Val Thr Glu Ala Gly Trp Pro Val Ser
    210                 215                 220

gga cca aca gtg gca caa gct gta gct tcc cct gaa aat gcc cgc tca       720

Gly Pro Thr Val Ala Gln Ala Val Ala Ser Pro Glu Asn Ala Arg Ser
225                 230                 235                 240

ttc tgg acc tcc gtt ggc tgc aac caa ctt ttc gat aag atc                762
Phe Trp Thr Ser Val Gly Cys Asn Gln Leu Phe Asp Lys Ile
                245                 250

<210>  4
<211>  254
<212>  PRT
<213>  Venturia inaequalis

<400>  4

Asn Tyr Gly Ser Thr Asp Ala Ser Gly Val Val Lys Asp Gln Ala Arg
1               5                   10                  15

Phe Glu Gln Glu Phe Ser Thr Ala Gln Asn Leu Val Gly Thr Ser Gly
            20                  25                  30

Phe Asn Ser Ala Arg Leu Tyr Thr Thr Ile Gln Gly Gly Thr Thr Asn
        35                  40                  45

Ser Pro Ile Ser Ala Ile Pro Ala Ala Ile Ser Thr Gln Thr Ser Leu
    50                  55                  60

Leu Leu Gly Ile Trp Thr Ser Ala Gly Gln Ala Ile Val Asp Asn Glu
65                  70                  75                  80

Ile Ala Ala Leu Lys Ala Ala Ile Ser Gln Tyr Gly Thr Ala Phe Thr
                85                  90                  95

Asp Leu Ile Val Ala Val Ser Val Gly Ser Glu Asp Leu Tyr Arg Asn
            100                 105                 110

Ser Gly Tyr Pro Gly Ala Ser Asp Pro Gly Pro Gly Ala Asn Pro Asp
        115                 120                 125

Val Leu Ala Asn Tyr Ile Gly Gln Val Lys Ala Ala Ile Ala Gly Thr
    130                 135                 140

Ser Ala Glu Gly Arg Leu Val Gly His Val Asp Thr Trp Thr Ala Phe
145                 150                 155                 160

Val Asn Ser Ser Asn Asn Ala Leu Ile Ser Ala Ala Asp Phe Leu Gly
                165                 170                 175

Val Asp Ala Tyr Pro Tyr Tyr Glu Ser Ala Asn Gly Asn Asp Ile Ser
            180                 185                 190

Asn Ala Ala Asn Leu Phe Ala Ser Ala Tyr Ser Gln Val Val Ala Val
        195                 200                 205

Ala Gln Gly Lys Pro Val Trp Val Thr Glu Ala Gly Trp Pro Val Ser

210                 215                 220

Gly Pro Thr Val Ala Gln Ala Val Ala Ser Pro Glu Asn Ala Arg Ser
225                 230                 235                 240

Phe Trp Thr Ser Val Gly Cys Asn Gln Leu Phe Asp Lys Ile
                245                 250

<210>  5
<211>  20
<212>  DNA
<213>  artificial

<220>
<223>  primer

<400>  5
atgttcgcaa aacttctcgc                                                   20

<210>  6
<211>  20
<212>  DNA
<213>  artificial

<220>
<223>  primer

<400>  6
actaggctgc gcggttggat                                                   20

## Beschreibung

Die vorliegende Anmeldung betrifft isolierte bifunktionale Polypeptide. Genauer betrifft die vorliegende Anmeldung Polypeptide mit einer Glucanaseaktivität und einer Proteaseaktivität kodiert durch die gleiche Aminosäuresequenz, wobei das Polypeptid ein Derivat eines natürlich vorkommenden Polypeptids mit Glucanase- und gleichzeitig einer Proteaseaktivität ist. In einem weiteren Aspekt werden Zusammensetzungen bereitgestellt dieses Polypeptid enthaltend. Schließlich richtet sich die vorliegende Anmeldung auf die Verwendung der erfindungsgemäßen Polypeptide in Wasch- oder Reinigungsmitteln, als antimikrobielles Mittel oder zum Aufschluss von tierischem, pflanzlichem und mikrobiellem Material sowie entsprechende Verfahren.

### Stand der Technik

Enzyme werden in verschiedensten Bereichen der roten, weißen und grünen Biotechnologie eingesetzt. So finden Enzyme heutzutage als waschaktive Substanzen in Reinigungsmitteln Anwendung, um dort Verschmutzungen auf biologischem Wege abzubauen. Dabei wird meist ein Cocktail verschiedener Enzymarten eingesetzt. Proteasen dienen zur Entfernung von Eiweißflecken und Pflanzenverfärbungen, Cellulasen minimieren eine Ausfransung des Gewebes und helfen bei der Entfernung des Grauschleiers. Lipasen werden zum Entfernen von Lipidbestandteilen eingesetzt. Amylasen werden gegen Stärkeflecken genutzt.

Enzyme finden aber auch in anderen Bereichen einen vielfältigen Einsatz. So werden Enzyme als Katalysatoren in verschiedensten Prozessen eingesetzt. In der grünen Biotechnologie werden Enzyme zur Herstellung von Bioethanol, zur Degradation von Polysacchariden usw. genutzt. Aber auch zur Herstellung von verschiedenen Lebensmittelprodukten und chemischen Verbindungen werden Enzyme eingesetzt. Dadurch können die Synthesewege häufig vereinfacht und kostengünstiger gestaltet werden.

Üblicherweise werden diese Enzyme rekombinant her- und bereitgestellt. Alternativ können diese Enzyme aus Mikroorganismen isoliert werden. Dieses ist allerdings kostenintensiver.

Enzyme weisen meist eine spezifische Enzymaktivität auf, z.B. als Lipase, Protease oder Glucanase. Das heißt, Enzyme zeigen üblicherweise eine einzige Enzymaktivität, d.h. es wird eine Reaktion durch dieses Enzym katalysiert. Enzyme, die mehr als eine Art an Aktivität aufzeigen, sind in der Natur selten. Meist ist die zweite Enzymaktivität auch wesentlich kleiner als die Hauptenzymaktivität. Üblicherweise werden zur Bereitstellung von Zusammensetzungen mit verschiedenen Enzymaktivitäten Cocktails dieser Enzyme eingesetzt. Dabei benötigen die Enzyme für die maximale Aktivität bestimmte Umgebungsbedingungen. Dem Fachmann sind die verschiedenen Parameter bekannt. Sie umfassen sowohl Umgebungsparameter, wie pH-Wert, Temperatur, Salzkonzentration usw. als auch die Konzentration der Ausgangsstoffe und der Endprodukte. Alternativ können mit Hilfe von Rekombinations-Techniken rekombinante Polypeptide hergestellt werden, die unterschiedliche Enzymaktivitäten aufweisen, sei es aus einer Spezies oder aus verschiedenen Spezies.

Ein großes Problem bei dem Einsatz von Cocktails mit verschiedenen Enzymen ist die Tatsache, dass die eingesetzten Enzyme üblicherweise verschiedene optimale Enzymaktivitätsbereiche aufweisen. Das heißt, während die Glucanase einen pH-Wert in einem ersten pH-Wert-Bereich aufweisen kann, kann die Protease ihre optimale Aktivität in einem hiervon verschiedenen zweiten pH-Wert-Bereich aufzeigen. Gleiches gilt für Temperaturbereiche und der Konzentration von Salzen etc. in der Umgebung.

In der Literatur sind verschiedene Zusammensetzungen aus Enzymen unterschiedlichen Ursprungs beschrieben. So wird in der DE 101 38 753 eine gentechnisch veränderte Amylase beschrieben, die aus Genabschnitten zweier verschiedener Bakterien zusammengesetzt ist und entsprechend als Hybridamylase bezeichnet wird. Aus der DD 296 407 A5 sind "Enzymkomplexe" beschrieben. Tatsächlich handelt es sich hierbei um Mischungen vieler verschiedener Enzyme, die aus der unterschiedlichen Spezien gewonnen werden.

Des Weiteren weisen die eingesetzten Enzyme unterschiedliche Temperaturstabilität auf. Die charakterisierten proteolytischen Enzyme (EC-Nummer 3.4.21) besitzen in ihrer Mehrheit ein Temperaturoptimum, daß zwischen 35 - 40°C liegt. Der optimale pH-Bereich dieser Enzymgruppe erstreckt sich vom sauren Bereich pH 2 bis zum alkalischen Bereich von pH11. Die Mehrzahl der Proteasen entfaltet eine optimale Wirkung im Bereich von pH 7 bis pH 9. Allerdings sind auch einige wenige Proteasen wie Subtilisin und Aqualysin aus thermophilen Bakterien *Thermococcus kodakaraensis* bzw. *Thermoaquaticus* bekannt, die ein Temperaturoptimum von 80°C haben. Enzyme, die in der Lage sind glykosidische Bindungen (EC-Nummer 3.2.1) zu spalten, verfügen über ein sehr breiten Temperaturbereich, der zwischen 40 und 65°C liegt. Der optimale pH-Wert für diese Enzymgruppe liegt im Gegensatz zu den Proteasen im leicht sauren bis neutralem Bereich zwischen pH 5 und pH 7.

Es besteht daher ein Problem entsprechend geeignete Zusammensetzungen, die unterschiedliche Enzymaktivitäten, z.B. sowohl Peptidase- als auch Glucanaseaktivität aufzeigen wobei beide Aktivitäten möglichst im Optimum genutzt werden können, bereitzustellen.

Der Pilz *Venturia inaequalis* ist im Volksmund als Apfelschorf bekannt. Ein Befall hiermit führt zu großen Ernteausfällen und Apfelschorf ist einer der wichtigsten Apfelbaumerkrankungen weltweit, die durch die Schlauchpilz *Venturia inaequalis* verursacht wird. Der Apfelschorf zeigt sich in den Blättern mit mattolivgrünen, später braun oder schwärzlichen Flecken, die zusammenfließen können und in der Folge größere Nekrosen bilden; dies führt zu einem vorzeitigen Blattverlust bei den Bäumen. Die Früchte weisen meist dunkler gefärbte Flecken auf, in denen meist sternenförmige Risse auftreten, die Fäulniserregern, wie Monilia, als Eintrittspforte dienen.

Von Hignett, R. C., Kerkham, D. S., J. Gen. Microbiol., 1967, 48, 269-275 wurde die Rolle eines extrazellulären Melanoproteins aus *Venturia inaequalis* bei der Wirtssuszeptibilität beschrieben. Vor kurzem wurde von Singh, P., et.al., Verlag der Zeitschrift für Naturforschung C, 2005, 60C, 109 - 115, die Aufreinigung eines extrazellulären Melanoproteins aus dem Pilz *Venturia inaequalis* beschrieben. Dort wird ein 36 kDa großes Protein dargestellt, das weiter charakterisiert wurde.

Aufgabe der vorliegenden Erfindung ist die Bereitstellung von neuen Enzymen, die verbesserte enzymatische Eigenschaften aufzeigen, insbesondere Enzyme, die eine unterschiedliche Enzymaktivität bei gleichen Optima in Bezug auf Temperatur, pH-Wert, usw., haben.

Eine weitere Aufgabe der vorliegenden Anmeldung ist die Bereitstellung von Zusammensetzungen, die eine hohe Reinigungsaktivität bzw. eine gute antimikrobielle Eigenschaft aufzeigen.

### Kurze Beschreibung der Erfindung

In einem ersten Aspekt richtet sich die vorliegende Erfindung auf Polypeptide, die eine enzymatische Bifunktionalität aufzeigen, nämlich sowohl eine Glucanaseaktivität als auch eine Proteaseaktivität haben, wobei dieses Polypeptid ein Derivat eines natürlich vorkommenden Polypeptids mit Glucanaseaktivität und gleichzeitiger Proteaseaktivität ist.

In einem weiteren Aspekt wird ein bifunktionales Polypeptid mit Glucanase-aktivität und Proteaseaktivität bereitgestellt, wobei das Polypeptid eines ist, wo dieser Bereich enzymatischer Aktivität nicht aus mindestens zwei verschiedenen Peptiden zusammengesetzt ist.

In einem weiteren Aspekt richtet sich die vorliegende Anmeldung auf Zusammensetzungen, insbesondere Reinigungszusammensetzungen, die die erfindungsgemäßen bifunktionalen Polypeptide mit zwei Enzymaktivitäten aufweisen.

Schließlich wird die Verwendung des erfindungsgemäßen Polypeptids in Wasch- oder Reinigungsmitteln, als antimikrobielles Mittel oder zum Aufschluss von tierischem, pflanzlichem und mikrobiellem Material beschrieben. In einem letzten Aspekt richtet sich die Anmeldung auf Verfahren zum Reinigen von Gegenständen bzw. Verfahren zum Abbau von tierischem, pflanzlichem und mikrobiellem Material, wobei diese Behandlung eine Behandlung mit dem erfindungsgemäßen Polypeptid mit zwei Enzymaktivitäten beinhaltet.

### Kurze Beschreibung der Abbildungen

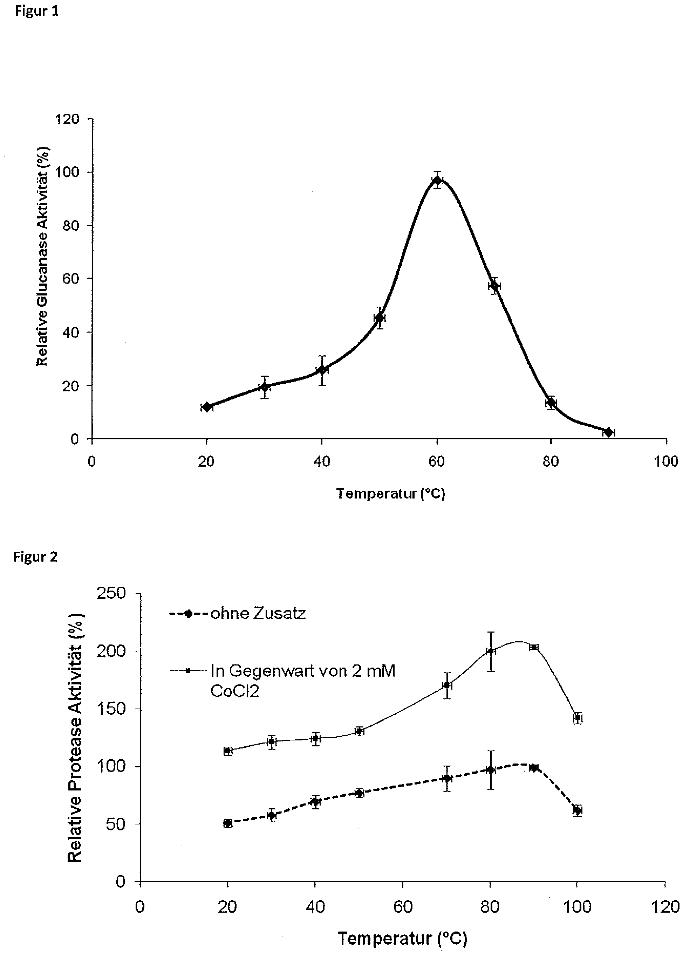

In der Figur 1 ist die Temperaturabhängigkeit der Glucanaseaktivität des erfindungsgemäßen bifunktionalen Polypeptids, vorliegend des rekombinanten Proteins aus *V. inaequalis,* dargestellt. Deutlich ist für die Glucanaseaktivität das Temperaturoptimum bei 60°C zu erkennen.

In der **Figur 2** ist die Temperaturabhängigkeit der Proteaseaktivität des rekombinanten Proteins aus *V. inaequalis* gezeigt. Zur Bestimmung des Temperaturoptimums wurde das rekombinante gereinigte Protein aus *V. inaequalis* eingesetzt. Durch die Anwesenheit von 2 mM CoCl₂ im Reaktionsansatz ist eine Erhöhung der Aktivität um 100 % zu beobachten.

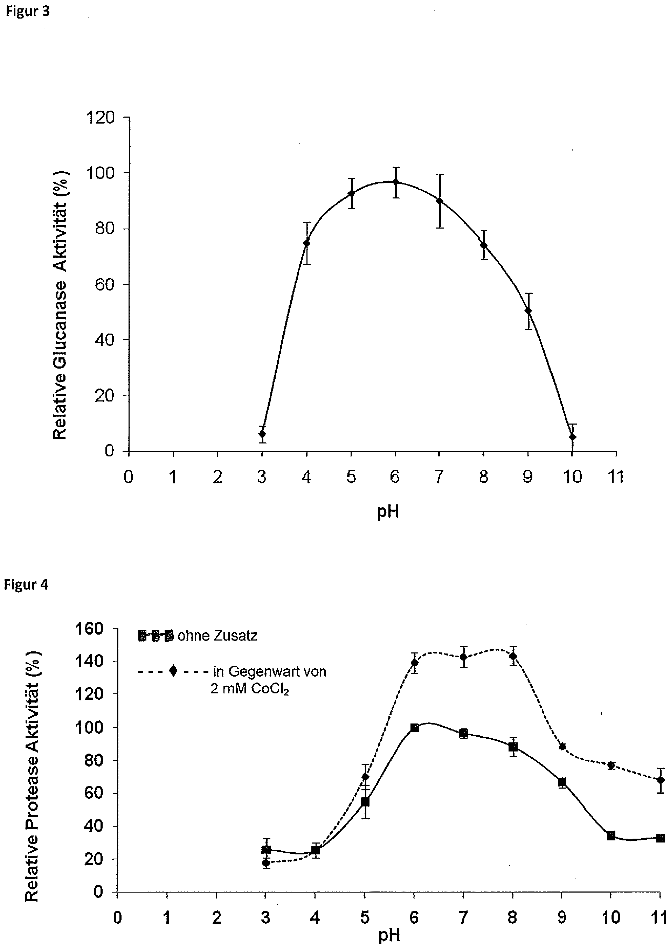

In der **Figur 3** ist die Glucanaseaktivität in Abhängigkeit des pH-Wertes für das erfindungsgemäße Polypeptid dargestellt.

In der **Figur 4** ist die pH-Abhängigkeit der Proteaseaktivität des erfindungsgemäßen Polypeptids dargestellt. Die Gegenwart von 2 mM Kobaltchlorid führt zu einer deutlichen Steigerung der Enzymaktivität (ca. 50%) im Bereich des pH-Optimums.

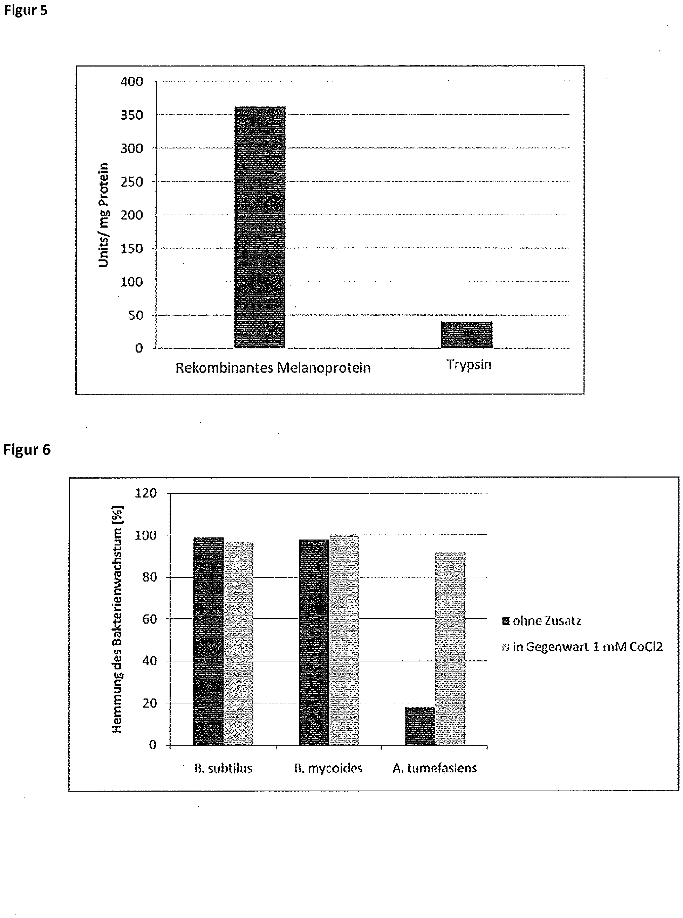

Die **Figur 5** zeigt die Ermittlung der Unit-Aktivität für das rekombinante Protein von V. inaequalis. Die Aktivitätsbestimmung erfolgte mit dem Substrat Azocasein. Die Reaktionsansätze wurden bei den jeweiligen pH-Optima von 6,0 und 8,3 sowie einer Inkubationstemperatur von 37°C° durchgeführt.

**Figur 6** zeigt den Effekt des rekombinanten Proteins aus V. inaequalis auf das Wachstum von Gram-positiven und Gram-negativen Bakterien in Abwesenheit und Gegenwart von 1 mM CoCl₂.

Die **Figur 7** stellt die Abhängigkeit der Proteaseaktivität des rekombinanten Proteins von V. inaequalis gegenüber verschiedenen Metallionen dar, Angaben in mM.

In der **Figur 8** ist die Abhängigkeit der Protease-Aktivität des rekombinanten Protein in Gegenwart von Melanin dargestellt. Für die Bestimmung der Aktivität wurden rekombinantes Protein mit der entsprechenden Menge Melanin vermischt und die Aktivität wie beschrieben bestimmt.

### Ausführliche Beschreibung der Erfindung

In einem ersten Aspekt wird ein Polypeptid bereitgestellt, das eine Bifunktionalität aufzeigt, nämlich eine Glucanaseaktivität und eine Protease-aktivität. Das bifunktionale Polypeptid ist ein Derivat eines natürlich vorkommenden Polypeptids mit Glucanaseaktivität und einer gleichzeitigen Protease-aktivität.

In einem weiteren Aspekt betrifft die vorliegende Erfindung ein isoliertes bifunktionales Polypeptid mit einer Glucanaseaktivität und einer Proteaseaktivität, wobei das Polypeptid eines ist, das nicht aus Bereichen mit enzymatischen Aktivitäten aus mindestens zwei verschiedenen Peptiden zusammengesetzt ist.

Das heißt, das erfindungsgemäße bifunktionale Polypeptid mit einer Glucanaseaktivität und einer Proteaseaktivität ist ein Derivat eines natürlich vorkommenden Polypeptids, d.h. eines natürlich vorkommenden Enzyms oder das natürlich vorkommende Enzym selbst in isolierter Form. In diesem Zusammenhang wird unter dem Ausdruck "Derivat eines natürlich vorkommenden Polypeptids" ein Polypeptid verstanden, bei dem, ableitend von der Aminosäuresequenz des natürlich vorkommenden Enzyms einige Aminosäurereste mit anderen Aminosäureresten ausgetauscht sein können, bevorzugt durch konservative Austausche, ohne dass die Glucanaseaktivität und gleichzeitige Proteaseaktivität des Polypeptids wesentlich verschlechtert sind, d.h. deren Enzymaktivität unter 50%, wie unter 70% der Enzymaktivität des natürlich vorkommenden Polypeptids fällt. Das natürlich vorkommende Polypeptid ist dabei eines isolierbar aus *Venturia inaequalis.* Insbesondere handelt es sich bei dem natürlich vorkommenden Polypeptid, d.h. ein natürlich vorkommendes bifunktionales Enzym mit einer Glucanaseaktivität und einer Proteaseaktivität, um ein Polypeptid gemäß der Aminosäuresequenz SEQ ID Nr. 2, z.B. kodiert durch die Seq. ID. Nr.1. Das erfindungsgemäße Derivat ist dabei insbesondere eines, das mindestens die Aminosäuresequenz gemäß SEQ ID Nr. 4, kodiert z.B. durch die Seq. ID Nr.3, umfasst.

Unter Aminosäuren oder Aminosäurereste im Sinne der Erfindung werden neben den 20 durch den genetischen Code bestimmten Aminosäuren auch die Aminosäuren, die durch Stop-Codons codiert werden können, wie z.B. Seleno-Cystein oder Pyrro-Lysin, verstanden. Außerdem sind alle bekannten Aminosäure- und Peptidderivate, wie z.B. glycosilierte, phosphorisierte, sulfatierte Aminosäuren/ Peptide, sowie L-isomere, D-isomere Aminosäuren eingeschlossen. Die Aminosäure- und Peptidderivate können durch posttranslationale Modifikation verändert sein. Auch ungewöhnliche Aminosäuren, die dem Fachmann bekannt sind, können in dem erfindungsgemäßen Polypeptid vorhanden sein.

Das bifunktionale Polypeptid zeichnet sich dadurch aus, dass es sowohl eine Glucanaseaktivität als auch eine Proteaseaktivität aufzeigt. Dieses Polypeptid ist dabei nicht ein zusammengesetztes Polypeptid, das enzymatische Aktivitäten (Glucanaseaktivität/ Proteaseaktivität) aus verschiedenen natürlichen Quellen und somit aus verschiedenen Peptiden zusammengesetzt aufweist. Vielmehr ist das Polypeptid eines, das sich von einem natürlich vorkommenden Polypeptid ableitet oder dieses ist. Dieses natürlich vorkommende Polypeptid ist dabei eines, das sowohl eine Glucanaseaktivität als auch eine Proteaseaktivität aufzeigt.

Insbesondere handelt es sich bei diesem natürlich vorkommenden Polypeptid um eines, das aus *Venturia inaequalis* isolierbar ist. Erfindungsgemäß ist daher das isolierte bifunktionale Polypeptid eines, das aus *Venturia inaequalis* isoliert wird oder, alternativ, dieses natürliche Polypeptid durch rekombinante Techniken in anderen Organismen hergestellt ist. Dem Fachmann sind geeignete rekombinante Techniken zur Herstellung von rekombinanten bifunktionalen Polypeptiden bekannt. Übliche Systeme schließen prokaryotische und eukaryotische Expressionssysteme ein, wie Expressionssysteme in Insektenzellen, in bakteriellen Zellexpressionssystemen, wie *E. coli,* aber auch Hefeexpressionszellsysteme und Expressionszellsysteme in eukaryotischen Zelllinien, insbesondere in Säugezelllinien, wie humane oder Rattenzelllinien.

Eine weitere Möglichkeit zur rekombinanten Herstellung des erfindungsgemäßen Polypeptids ist die Expression in gentechnisch veränderten Pflanzen.

Vorliegend ist insbesondere eine Expression in bakteriellen Zellexpressions-systemen bevorzugt, um die notwendigen großen Mengen an Enzymen rekombinant bereitzustellen.

Bevorzugt ist das isolierte bifunktionale Polypeptid eines, das die Protease-aktivität innerhalb des Aminosäureabschnitts mit der Glucanaseaktivität aufzeigt. D.h., bevorzugt zeichnet sich das isolierte bifunktionale Polypeptid dadurch aus, das diese Proteaseaktivität in dem Aminosäureabschnitt mit der Glucanaseaktivität lokalisiert ist. Insbesondere weist dieses bifunktionale Polypeptid einen Aminosäureabschnitt auf, der eine Glucanaseaktivität und innerhalb dieser Glucanaseaktivität weiterhin eine Proteaseaktivität aufzeigt, insbesondere ist der Aminosäureabschnitt einer mit einer Aminosäuresequenz gemäß SEQ ID Nr. 4.

Das erfindungsgemäße isolierte bifunktionale Polypeptid mit Glucanaseaktivität und Proteaseaktivität ist dabei insbesondere eines, das eine Glucanaseaktivität mit einem Temperaturoptimum von 60°C aufzeigt. Weiterhin ist das erfindungsgemäße Polypeptid eines, das eine Proteaseaktivität stabil bis zu 90°C aufzeigt.

D.h., das erfindungsgemäße Polypeptid mit bifunktionaler Enzymaktivität ist eines, das bevorzugt eine sehr hohe Temperaturstabilität der Enzymaktivität hat. So kann die Glucanaseaktivität ein Temperaturoptimum bei 60°C aufweisen, die Proteaseaktivität kann ihr Optimum erst bei 90°C erreichen. Bevorzugt ist insbesondere, dass die Proteaseaktivität bei einer Temperatur von 100°C noch bei 90% liegt, während bereits bei 20°C schon eine Proteaseaktivität von mindestens 60% der maximalen Proteaseaktivität erreicht wird. Das erfindungsgemäße bifunktionale Polypeptid zeichnet sich somit durch einen großen Temperaturbereich aus, in dem die Proteaseaktivität vorliegt. Gleiches gilt für die Glucanase-aktivität. Die Glucanaseaktivität bei einem Temperaturbereich von über 45°C bis über 70°C weist eine Glucanaseaktivität von >40% auf. Die optimale Temperatur ist 60°C.

Weiterhin ist in einer bevorzugten Ausführungsform das bifunktionale Polypeptid, das ein Derivat des natürlich vorkommenden Polypeptids mit Glucanaseaktivität und gleichzeitig einer Proteaseaktivität bzw. das natürlich vorkommende Polypeptid selbst ist, **dadurch gekennzeichnet, dass** es eine Glucanaseaktivität von mindestens 50% über einen Bereich von pH 4 bis pH 8 aufweist und/oder eine Proteaseaktivität von mindestens 40% über einen Bereich von pH 3 bis pH 11 aufweist.

D.h., im Gegensatz zu vielen bekannten Proteasen zeigt die erfindungsgemäße Protease auch im leicht sauren pH-Bereich bereits optimale Enzymaktivität auf. Das erfindungsgemäße bifunktionale Polypeptid ist dabei bevorzugt eines, das ein pH-Optimum im leicht sauren Bereich, wie bei einem pH von 6 aufzeigt. Auch das pH-Optimum der Glucanaseaktivität des erfindungsgemäßen bifunktionalen Polypeptids ist bei ca. pH 6. Dadurch ist es möglich sowohl Proteaseals auch Glucanaseaktivität bei einem gleichen pH-Wert einzusetzen und zu nutzen.

In einem weiteren Aspekt zeichnet sich das erfindungsgemäße bifunktionale Polypeptid dadurch aus, dass es sowohl 1,3, 1,4 als auch 1,6- glykosidische Bindungen spaltet. D.h., die erfindungsgemäße Glucanaseaktivität erlaubt die Spaltung verschiedener glycosidischer Bindungen und ist daher besonders gut geeignet entsprechende glycosidische Strukturen aufzubrechen und abzubauen, z.B. im Bereich der Reinigung oder dem Abbau von biologischem Material, z.B. in der Biomasse- oder Bioethanolherstellung.

Durch diese Eigenschaft, verschiedenste glycosidische Bindungen zu spalten, in Kombination mit der Eigenschaft der proteolytischen Enzymaktivität, ist ein umfangreicher Einsatz dieses Polypeptids möglich.

In einer bevorzugten Ausführungsform ist das bifunktionale Polypeptid insbesondere eines, das bei Zugabe von Melanin beide Enzymaktivitäten aufweist. Es zeigte sich, dass dieses rekombinante Protein, im Folgenden auch als Melanoprotein bezeichnet, weiterhin zusätzlich freies Melanin binden kann, um mit diesem ein Holoprotein auszubilden. Durch Binden dieses freien Melanins kann eine deutliche Steigerung der Enzymaktivität erzielt werden. Erfindungsgemäß ist dabei insbesondere bevorzugt das bifunktionale Polypeptid ein Melanoprotein, das bevorzugt mit freiem Melanin gebunden ist, um ein Holo-protein auszubilden.

In einer weiteren bevorzugten Ausführungsform ist das bifunktionale Polypeptid eines, das Kobaltionen binden kann und sich dadurch auszeichnet, dass bei Bindung von Kobaltionen eine verbesserte Temperaturstabilität, eine erhöhte Enzymaktivität und/oder eine Erweiterung des pH-Optimums erzielt wird.

Erfindungsgemäß konnte dargestellt werden, dass das Vorhandensein von Kobalt z.B. in Form eines Kobaltsalzes in der Zusammensetzung, die das erfindungsgemäße bifunktionale Polypeptid enthält, die Enzymaktivität des erfindungsgemäßen bifunktionalen Polypeptids erhöht und auch das pH-Optimum der Enzymaktivität erweitert ist. In einer bevorzugten Ausführungsform weist daher die erfindungsgemäße Zusammensetzung enthaltend das erfindungsgemäße Polypeptid mit bifunktionaler Enzymaktivität weiterhin Kobalt auf.

Es zeigte sich weiterhin, dass das erfindungsgemäße Polypeptid eine höhere spezifische Proteaseaktivität für z.B. das künstliche Substrat Azocasein im Vergleich zu bekannten kommerziell erhältlichen Proteasen aufzeigt. So zeigt das erfindungsgemäße Polypeptid mit Proteaseaktivität eine entsprechende spezifische Aktivität auf, die um den Faktor 9 größer ist als die von Trypsin, einer bekannten und häufig eingesetzten kommerziellen Protease. Aufgrund dieser ungewöhnlich hohen Enzymaktivität kann die Menge an Enzym in den verschiedenen biotechnologischen Prozessen, wo dieses Enzym eingesetzt wird, deutlich verringert werden. Das führt natürlich zu deutlich gesenkten Kosten.

Das erfindungsgemäße Enzym hat dabei bevorzugt eine Aktivität der Glucanase von mindestens 10 Units/mg Protein/min. Die Proteaseaktivität ist bevorzugt mindestens 100 Units/mg Protein/min, wie mindestens 200 U/mg Protein/min. Insbesondere ist die Proteaseaktivität größer 300 U/mg Protein/min.

Auch das erfindungsgemäße Polypeptid eignet sich sehr gut zur rekombinanten Herstellung. D.h., aufgrund der vorliegenden ungewöhnlichen Temperaturstabilität kann ein rekombinantes Protein einfach durch Hitzebehandlung (30 Min bei 70°C) aus dem Wirtsorganismus, z.B. *E. coli,* gewonnen werden.

Aufgrund dieser guten Stabilität bei hohen Temperaturen ist das erfindungsgemäße Polypeptid besonders gut zur rekombinanten Gewinnung mittels bekannten Expressionssystemen geeignet.

In einem weiteren Aspekt richtet sich die vorliegende Anmeldung auf Zusammensetzungen, die das erfindungsgemäße Polypeptid enthalten. Bei diesen Zusammensetzungen handelt es sich insbesondere um Reinigungszusammensetzungen, aber auch antimikrobielle Zusammensetzungen oder Zusammensetzung zum Abbau von tierischem, pflanzlichem oder mikrobiellem Material.

Die erfindungsgemäße Zusammensetzung enthält dabei bevorzugt weiterhin Melanin zur Ausbildung von Polypeptid-Melanin-Holoproteinen. Alternativ oder zusätzlich enthält die Zusammensetzung weiterhin Kobalt, z.B. in Form von Kobaltsalzen. Als geeignetes Salz kann z.B. Kobaltchlorid eingesetzt werden. Die zugeführte Menge an Kobaltchlorid kann dabei in einem Bereich von 0,25 mM bis 3 mM, bevorzugt 1 bis 3 mM, wie 2 mM Kobaltchlorid, liegen. Alternativ kann ein Zinksalz eingesetzt werden. Das Salz Zinkchlorid zeigt in einem Konzentrationsbereich von 1 mM bis 5 mM eine gesteigerte Proteaseaktivität. Das Maximum der Aktivität liegt bei 4 mM.

Es zeigte sich, dass das erfindungsgemäße Polypeptid die Vermehrung von sowohl von gram-positiven Bakterien, z.B. *Bacillus subtilis* oder *Bacillus mycoi*des, aber auch von gram-negativen Bakterien hemmt. Entsprechend ist das Polypeptid als Bestandteil von antimikrobiellen Zusammensetzungen geeignet und kann entsprechend in medizinischen Anwendungen eingesetzt werden.

Die erfindungsgemäße Zusammensetzung enthaltend das erfindungsgemäße Polypeptid hat somit sowohl reinigende Wirkung als auch antimikrobielle Wirkung und kann z.B. zur Dekontamination von Oberflächen eingesetzt werden.

Ein wesentlicher Einsatzbereich der Zusammensetzungen bzw. des erfindungsgemäßen Polypeptids liegt aber insbesondere auf dem Gebiet der Wasch- und Spülmittel. Bereits heute werden unterschiedlichste Enzyme in Wasch- und Reinigungsmitteln eingesetzt. Sie erlauben Verschmutzungen effizient und unter Vermeidung von gesundheitsgefährdenden Chemikalien zu reinigen. Dabei sind die Enzyme für verschiedenste Aufgaben aufgrund ihrer Enzymaktivität geeignet. Sie können sowohl Fettbestandteile als auch Kohlehydratverschmutzungen sowie Eiweißverschmutzungen entfernen. Das erfindungsgemäße Polypeptid bzw. die erfindungsgemäße Zusammensetzung ist daher insbesondere in der Verwendung als Wasch- oder Reinigungsmittel, als antimikrobielles Mittel oder zum Aufschluss von tierischem, pflanzlichem oder mikrobiellem Material geeignet.

Die erfindungsgemäßen Polypeptide zeigen enzymatische Aktivität gegenüber sowohl Poly- als auch Oligosacchariden, wie Amylose, Zellulose, Glucan, Guaran usw. Aufgrund ihrer breiten Substratspezifikation, d.h. der Fähigkeit 1,3-, 1,4- und 1,6-glycosidische Bindungen aufzulösen, eignen sich die erfindungsgemäßen Polypeptide hervorragend als Bestandteil von Wasch- und Reinigungsmitteln. Weiterhin kann das Polypeptid gleichzeitig aufgrund seiner protolytischen Aktivität Proteine, z.B. Eiweiße, abbauen.

Ein weiteres Anwendungsgebiet der erfindungsgemäßen Zusammensetzung bzw. der erfindungsgemäßen Polypeptide ist der Abbau von tierischem, pflanzlichem oder mikrobiellem Bereich in biotechnologischen Anwendungen. Diese biotechnologischen Anwendungen umfassend sowohl Lebensmittel als auch Biomasse, wie z.B. Bioethanolproduktion.

Schließlich richtet sich die vorliegende Erfindung auf Verfahren zum Reinigen und Säubern von Gegenständen umfassend den Schritt des Behandelns dieser Gegenstände mit einer erfindungsgemäßen Zusammensetzung bzw. einem erfindungsgemäßen Polypeptid. In einem weiteren Aspekt betrifft die Anmeldung ein Verfahren zum Abbau von Pflanzenmaterial aber auch von tierischem oder mikrobiellem Material, wobei dieses Verfahren insbesondere eines im Bereich der Lebensmittelproduktion oder im Bereich der grünen Biotechnologie, z.B. der Biomasse- oder Bioethanolherstellung, betrifft. Das erfindungsgemäße Verfahren umfasst dabei den Schritt des Behandelns dieses Materials mit einer erfindungsgemäßen Zusammensetzung bzw. einem erfindungsgemäßen Polypeptid.

Im Folgenden wird die Erfindung mit Hilfe der Beispiele näher erläutert, ohne diese auf die Beispiele zu beschränken.

### Beispiele

### 1. Herstellung des rekombinanten Melanoproteins

Für die rekombinante Herstellung des Melanoproteins wurde das Gen des Melanoproteins aus *Venturia inaequalis* mit sequenzspezifischen Primern (Seq. ID. Nr. 5 und 6) durch die Polymerasekettenreaktion amplifiziert, in den Vektor pET32 ligiert und in den Bakterienstamm *E.coli* BL21 codon plus kloniert. Die Überexpression erfolgt durch Anzucht des entsprechenden *E.coli* Klons in Luria Broth Medium bei 37°C. Bei Erreichen einer Zelldichte von OD₆₀₀ erfolgte die Induktion der Genexpression durch Zugabe von 1 mM Isopropyl-β-D-thiogalactopyranosid. Nach weiteren drei Stunden der Bakterienanzucht wurden die Bakterienzellen durch Zentrifugation für 20 Minuten bei 5000 xg und 4°C sedimentiert. Anschließend wird das Sediment in 50 mM Natriumphosphat Puffer pH 7,0 aufgenommen und auf eine Zelldichte von 100 µl gepacktes Zellvolumen pro Milliliter eingestellt. Danach folgte eine einmalige Passage durch eine French Presse bei 1200 bar. Der Extrakt wird für 30 Minuten bei 16000 xg zentrifugiert. Der Überstand enthält das rekombinante Melanoprotein und kann für die weitere Aufreinigung auf eine Nickel-Affinitätssäule gegeben werden. Alternativ kann eine Aufreinigung des rekombinanten Melanoproteins durch eine Wärmebehandlung von 30 Minuten bei 65°C erfolgen.

### 2. Charakterisierung des Melanoproteins aus V. inaequalis

### a) Bestimmung Enzymaktivität

### Protease-Aktivität:

Die Proteaseaktivität wurde mit dem artifiziellen Substrat Azocasein bestimmt. Für die Proteasebestimmung wurden 2,5 mL 2,5% Azocasein Lösung mit 1,5 mL 0,5% Natriumbicarbonat Puffer pH 8,3 versetzt und zu 1 ml der zu bestimmenden Enzymlösung gegeben (1 mg/ml rekombinantes Protein). Nachfolgend folgt eine Inkubation für 30 Minuten. Im Anschluss daran wird zum Stoppen der Reaktion 4 mL 5 % Trichloressigsäure hinzugefügt, unverzüglich gemischt und für 5 Minuten bei 10000 xg abzentrifugiert. Danach erfolgt die Entnahme von 1 ml Überstand und versetzt diesen mit 3 mL 500 mM Natronlauge. Nach der Mischung erfolgt nun die Bestimmung der optischen Dichte bei 440 nm.

### Glucanase-Aktivität

Die Bestimmung der Glucanase-Aktivität erfolgt mit der Kongo Rot Methode. Hierzu erfolgte eine Inkubation des rekombinanten Proteins mit 5 mg/mL Glucan in Gegenwart von 50 mM Natrium-Phosphat Puffer pH 6,0 bei 40°C und 20 µL 0,1% Kongo Rot Lösung pro Milliliter Reaktionsansatz für 30 Minuten. Durch Zugabe von 10 µL 2 M Schwefelsäure wird der Reaktionsansatz gestoppt und durch weitere 10 µL 2 M Natronlauge neutralisiert. Durch hinzufügen von 400 µM 100% Essigsäure und 600 µL 100% Aceton erfolgte die Präzipitation des nichtumgesetzten Glucans nach erfolgter Zentrifugation von fünf Minuten bei 10000 xg. Durch Messung der optischen Dichte bei 520 nm konnte die Glucanase-Aktivität bestimmt werden.

### b) Bestimmung pH-Optimum,

Die Bestimmung der pH- Optima der Glucanase- und der Protease-Aktivität erfolgte nach den oben beschriebenen Methoden. Es wurde für jeden Messpunkt ein Puffer mit entsprechendem pH-Wert eingesetzt. Um Autohydrolysen der Substrate auszuschließen, wurden Kontrollansätze zu jedem pH-Wert ohne Enzymzugabe durchgeführt.

### c) Bestimmung Temperaturoptimum,

Die Bestimmung der Temperatur-Optima der Glucanase- und der Protease-Aktivität erfolgte nach den oben beschriebenen Methoden. Um Autohydrolysen der Substrate auszuschließen, wurden Kontrollansätze zu jedem TemperaturWert ohne Enzymzugabe durchgeführt.

### 3. Vergleich Proteaseaktivität mit Trypsin

Der Vergleich der Proteaseaktivitäten zwischen dem rekombinanten Melanoprotein und dem Enzym Trypsin erfolgte mit dem künstlichen Substrat Azocasein (siehe oben). Hierzu wurden 10 mg Trypsin bzw. 1 mg rekombinantes Melanoproteins gelöst in 1ml Puffer eingesetzt. Für Trypsin wurde ein Puffer mit einem pH-Wert von 8,3 gewählt, für das Melanoprotein der pH-Wert von 6,0. Beide Reaktionsansätze wurden für 30 Minuten bei 37°C inkubiert und im Anschluss wie oben beschrieben bestimmt.

### 4. Bestimmung der antimikrobiellen Eigenschaft

Zur Bestimmung der antimikrobiellen Eigenschaften wurden zunächst die Bakterien *Bacillus subtilus, Bacillus mycoides* und *Agrobacterium tumefasiens* bis zu einer optischen Dichte von 0,6 (1 x 10⁸ Zellen pro 1,5 mL) in Luria Broth Medium angezogen. Dieser Kultur wurden 100 µL Zellsuspension (1 x 10⁷ Zellen) entnommen, mit 50 µg rekombinantem Melanoprotein versetzt, auf ein Endvolumen von 1,5 mL mit Luria Broth Medium aufgefüllt und für weitere 24 Stunden bei 28°C inkubiert. Nachfolgend wird der vollständige Ansatz in 5 mL M9 Medium mit 5 mM HEPES pH 7,0, 0,025% Glucose und 0,5 mM Asparagin überführt und für weitere acht Stunden bei 28°C inkubiert. Die Hemmung des Bakterienwachstums wird durch die Bestimmung der optischen Dichte bei 600 nm bestimmt. Zur Untersuchung des Einflusses von Kobaltchlorid erfolgte der Ansatz in Gegenwart von 1 mM Kobaltchlorid.

### 5. Interaktion mit freiem Melanin

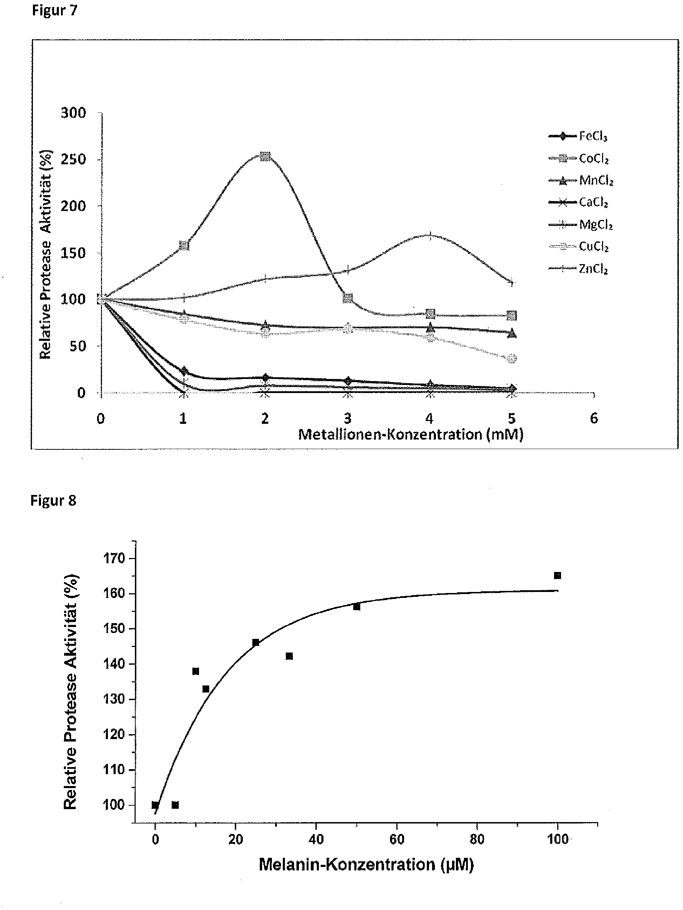

Zur Untersuchung der Interaktion mit löslichem Melanin wurden 30 µg rekombinantes Melanoprotein in 100 µL gelöst und mit der entsprechenden Menge Melanin versetzt. Nachfolgend wurde der Ansatz für 20 Minuten bei Raumtemperatur inkubiert und anschließend zur Bestimmung der Proteaseaktivität (wie oben beschrieben) eingesetzt. Die Zugabe von Melanin zum rekombinanten Melanoprotein aus *Venturia inaequalis* führt zu einer deutlichen Steigerung der Protease-Aktivität (siehe Figur 8). Außerdem führt die Anwesenheit von Melanin in Gegenwart von 2 mM Kobaltchlorid zur Ausbildung von hochmolekularen Strukturen (209, 536, 973, 1490, 2946, 5126, 7057 kD).

### 6. Enzymaktivität

Bei einem pH-Wert von 6,0 und einer Temperatur von 60°C zeigt das beschriebene Enzym eine Glucanaseaktivität von 14.7 Units/mg Protein/min auf. Dabei ist eine Unit Glucanase definiert als: 1 Unit Glucanaseaktivität produziert 1µmol Glucose pro Minute.

Die Proteaseaktivität des beschriebenen Enzyms ist bei einem pH-Wert von 7,0 und einer Temperatur von 90°C 367 Units/mg Protein/min. Dabei ist eine Unit Protease definiert als 1 Unit Protease spaltet 1 µm Azocasein pro Minute.

## Patentansprüche

1. Isoliertes, bifunktionales Polypeptid mit einer Glucanaseaktivität und einer Proteaseaktivität, wobei das Polypeptid ein natürlich vorkommendes Polypeptid oder ein Derivat eines natürlich vorkommenden Polypeptids mit Glucanaseaktivität und gleichzeitig einer Proteaseaktivität ist.

2. Isoliertes, bifunktionales Polypeptid mit einer Glucanaseaktivität und einer Proteaseaktivität, wobei das Polypeptid eines ist, das nicht aus Bereichen mit enzymatischen Aktivitäten aus mindestens zwei verschiedenen Peptiden zusammengesetzt ist.

3. Isoliertes, bifunktionales Polypeptid nach Anspruch 1 oder 2, isolierbar aus *Venturia inaequalis* oder wobei dieses rekombinant hergestellt ist.

4. Isoliertes, bifunktionales Polypeptid nach einem der vorherigen Ansprüche, **dadurch gekennzeichnet, daß** es weiterhin Melanin enthält.

5. Isoliertes, bifunktionales Polypeptid nach einem der vorherigen Ansprüche, **dadurch gekennzeichnet, daß** die Proteaseaktivität innerhalb des Aminosäureabschnitts mit der Glucanaseaktivität lokalisiert ist.

6. Isoliertes, bifunktionales Polypeptid nach einem der vorherigen Ansprüche, **dadurch gekennzeichnet, daß** die Glucanaseaktivität bis zu einer Temperatur von 60°C stabil ist und/oder die Proteaseaktivität bis zu 90°C stabil ist.

7. Isoliertes, bifunktionales Polypeptid nach einem der vorherigen Ansprüche, **dadurch gekennzeichnet, daß** dieses eine Glucanaseaktivität von mindestens 50 % über einen Bereich von pH 4 bis pH 8 aufweist und/oder eine Proteaseaktivität von mindestens 40 % über einen Bereich von pH 3 bis pH 11 aufweist, insbesondere daß das pH-Optimum der Glucanaseaktivität bei ca. pH 6 liegt und/oder das pH-Optimum der Proteaseaktivität bei ca. pH 6 liegt.

8. Isoliertes, bifunktionales Polypeptid nach einem der vorherigen Ansprüche, **dadurch gekennzeichnet, dass** es eine 1,3-, 1,4-, und 1,6-glycosidische Aktivität aufzeigt.

9. Isoliertes, bifunktionales Polypeptid nach einem der vorherigen Ansprüche, **dadurch gekennzeichnet, daß** es eine antimikrobielle Aktivität zumindest gegenüber gram-positiven Bakterien aufzeigt.

10. Isoliertes, bifunktionales Polypeptid nach einem der vorherigen Ansprüche, **dadurch gekennzeichnet, daß** es Kobaltionen oder Zinkionen binden kann und bevorzugt bei Anwesenheit von Kobalt oder Zink eine verbesserte Temperaturstabilität, eine erhöhte Aktivität und/oder eine Erweiterung des pH Optimums aufweist.

11. Isoliertes, bifunktionales Polypeptid nach einem der Ansprüche 1 bis 10, **dadurch gekennzeichnet, daß** es ein Polypeptid mit der Seq. ID. Nr. 2 ist oder ein Derivat hiervon umfassend mindestens die Aminosäuresequenz gemäß Seq. ID. Nr. 4.

12. Zusammensetzung, insbesondere Reinigungszusammensetzung oder antimikrobielle Zusammensetzung enthaltend ein Polypeptid nach einem der Ansprüche 1 bis 11.

13. Verwendung eines Polypeptids nach einem der Ansprüche 1 bis 11 als Wasch- oder Reinigungsmittel, als antimikrobielles Mittel, oder zum Aufschluss vom tierischen, pflanzlichen und mikrobiellen Materials.

14. Verfahren zum Reinigen von Gegenständen umfassend den Schritt des Behandelns dieses Gegenstandes mit einer Zusammensetzung enthaltend ein Polypeptid gemäß einem der Ansprüche 1 bis 11.

15. Verfahren zum Abbau von Pflanzenmaterial, insbesondere Pflanzenmaterial im Bereich der Lebensmittelproduktion und biotechnologischen Verfahren, umfassend den Schritt des Behandelns dieses Pflanzenmaterials mit einem Polypeptid nach einem der Ansprüche 1 bis 11.
